# EUROPÄISCHE PATENTSCHRIFT

(11) **EP 4 391 978 B1**
(45) Veröffentlichungstag und Bekanntmachung des Hinweises auf die Patenterteilung: **01.10.2025**
(21) Anmeldenummer: 22786279.4
(22) Anmeldetag: 23.08.2022
(51) Int. Cl.: A61F 5/01

(54) **EINSTELLBARES ORTHESENGELENK ZUR KONTROLLIERTEN BEWEGUNG UND/ODER FIXIERUNG EINER HAND SOWIE ORTHESE MIT EINEM DERARTIGEN ORTHESENGELENK**
ADJUSTABLE ORTHOSIS JOINT FOR THE CONTROLLED MOVEMENT AND/OR FIXATION OF A HAND, AND ORTHOSIS HAVING AN ORTHOSIS JOINT OF THIS KIND
ARTICULATION D'ORTHÈSE RÉGLABLE POUR LA MISE EN MOUVEMENT ET/OU L'IMMOBILISATION CONTRÔLÉES D'UNE MAIN ET ORTHÈSE POURVUE D'UNE TELLE ARTICULATION D'ORTHÈSE

(30) Priorität: 23.08.2021 DE 102021121819
(43) Veröffentlichungstag der Anmeldung: 03.07.2024
(73) Patentinhaber: Kimebo UG (Haftungsbeschränkt), 80331 München (DE)
(72) Erfinder: Klimmer, Uwe, 80331 München (DE)
(74) Vertreter: Heilein, Ernst-Peter
(86) Internationale Anmeldenummer: PCT/DE2022/100632
(87) Internationale Veröffentlichungsnummer: WO 2023/025354

(56) Entgegenhaltungen:
- DE-A1- 102019 130 404
- US-A1- 2004 019 306
- US-A1- 2011 282 253

## Beschreibung

Die vorliegende Erfindung betrifft ein einstellbares Orthesengelenk zur kontrollierten Bewegung und/oder Fixierung einer Hand eines Patienten, wenigstens umfassend einen ersten Schwenkhebel zur Ausführung einer Extensions- und/oder Flexionsbewegung der Hand und einen, am ersten Schwenkhebel schwenkbar angeordneten, zweiten Schwenkhebel zur Ausführung einer ulnaren und/oder radialen Deviation der Hand, sowie eine Orthese zur kontrollierten Bewegung und/oder Fixierung einer Hand oder einer Hand und eines Unterarms eines Patienten mit einem solchen Orthesengelenk.

Im Rahmen des Rehabilitationsprozesses nach einer Handverletzung muss die betroffene Hand bzw. der Unterarm des Patienten in vielen Fällen eine gewisse Zeit lang ruhiggestellt werden. Die Ruhigstellung erfolgt dabei gewöhnlich mit Hand-/Unterarm-Orthesen, welche Hand und Unterarm in einer bestimmten Position fixieren und nach erfolgter Fixierung keinerlei Bewegung der besagten Gliedmaßen mehr zulassen. Eine für die Fixierung der Hand bzw. des Unterarms ausgelegte Orthese wird beispielsweise in der US 2021/014 56 20 A1 offenbart. Die darin beschriebene Schiene besteht aus wenigstens zwei justierbaren Haltemitteln, welche auf die palmare bzw. dorsale Oberseite des Unterarms Druck ausüben können. Durch kontrollierte Ausrichtung besagter Haltemitte zueinander und anschließender Fixierung in einer gewünschten Position, lassen sich die Knochenfragmente eines Unterarmbruchs im Rahmen einer Frakturbehandlung stabilisieren. Dabei werden allerdings auch zwangsläufig Gelenke der Hand und/oder des Unterarms ruhiggestellt, die gar nicht von der Verletzung betroffen sind. Je nach Tragedauer einer solchen Orthese kann dies nachteilig zu sog. Sekundär-Versteifungen der von der Verletzung nicht betroffenen Gelenke führen, deren zusätzliche Mobilisation nach Ende des Heilungsverlaufs unnötige Kosten wegen einer verlängerten Rehabilitationszeit und somit längerer Arbeitsausfallzeiten des Patienten verursacht. Bei komplexen Verletzungen der Hand bzw. des Unterarms kann es zudem wünschenswert sein, einzelne, in ihrem Heilungsprozess weiter fortgeschrittene Gelenke bereits kontrolliert zu bewegen und dabei gleichzeitig noch stärker geschädigte Gelenke weiterhin zu fixieren. Unter einer "kontrollierten Bewegung" soll in diesem Zusammenhang insbesondere eine, in ihrem Bewegungswinkel eingeschränkt, geführte Bewegung verstanden werden. In der Physiotherapie und Orthopädie werden hierzu unter anderem oftmals Orthesen eingesetzt, welche Gliedmaßen aufnehmen und die Bewegung eines bestimmten Gelenks in einem vorgegebenen Winkelbereich freigeben, so dass der Patient die Bewegung selbstständig und sicher ausführen kann. Der Winkelbereich kann dann, je nach dem Trainings- bzw. Gesundheitszustand des Patienten, nach und nach erweitert werden, um schließlich den ursprünglichen Bewegungsradius wiederzuerlangen.

Aus dem Stand der Technik sind solche "range-of-motion" (ROM)-Vorrichtungen insbesondere für Knie-Orthesen bzw. für die Beuge- und Streckbewegung des Knies bekannt. Die EP 0 546 331 A1 und die EP 0 832 624 A2 offenbaren beispielsweise Orthesengelenke mit einstellbarem Bewegungsumfang bezogen auf eine Bewegung in einer Ebene, welche vorzugsweise in einer Knie-Orthese zum Einsatz kommen können. Aus der US 7,081,102 B1 ist zudem eine Karpaltunnel-Stütze mit Gelenk bekannt, welche eine eingeschränkte, radiale und/oder ulnare Deviation der Hand des Benutzers erlaubt. Eine ähnliche Vorrichtung zur kontrollierten Deviation der Hand wird in der CN 2 10 843 695 U beschrieben.

Das menschliche Handgelenk zeichnet sich allerdings durch einen komplexeren, "mehrdimensionalen" Bewegungsumfang aus, welcher durch eine Kombination aus einer Extensions-/Flexions-Bewegung (Beuge- und Streckbewegung in einer Ebene senkrecht zur Handfläche) und aus einer ulnaren bzw. radialen Deviation (Wischbewegung in der Ebene der Handfläche) beschrieben werden kann. Die aus dem Stand der Technik bekannten, in ihrem Bewegungsumfang einstellbaren Orthesengelenke und ROM-Vorrichtungen lassen die Kontrolle einer derartigen mehrdimensionalen Bewegung nicht zu, wie beispielsweise bei der in der US 2004/001 93 06 A1 beschriebenen Vorrichtung, bzw. benötigen mehrere, getrennte Gelenke. Exemplarisch seien hierzu die DE 10 2019 130 404 A1 bzw. WO 2021/093 919 A2 sowie die US 2011/028 22 53 A1 genannt.

Hiervon ausgehend liegt der vorliegenden Erfindung die Aufgabe zugrunde, ein gegenüber dem Stand der Technik verbessertes Orthesengelenk, insbesondere für eine Hand-Orthese, bereitzustellen, welches sowohl die Kontrolle einer Extensions-/Flexionsbewegung als auch einer ulnaren und/oder radialen Deviation der Hand eines Patienten ermöglicht und dabei möglichst kompakt gebaut und dennoch robust ausgestaltet ist.

Diese Aufgabe wird durch ein einstellbares Orthesengelenk mit den Merkmalen des unabhängigen Patentanspruchs 1 bzw. durch eine Orthese mit den Merkmalen des nebengeordneten Patentanspruchs 10 gelöst.

Das erfindungsgemäße Orthesengelenk zeichnet sich gegenüber Orthesengelenken des Stands der Technik dadurch aus, dass das Orthesengelenk ein Befestigungselement zur schwenkbaren Befestigung des ersten Schwenkhebels; ein erstes, oberes Winkeleinstellelement, welches bei Verwendung bezüglich eines Handrückens oberhalb des Befestigungselements, um eine Drehachse drehbar, an dem Befestigungselement angeordnet ist; und ein zweites, unteres Winkeleinstellelement, welches bei Verwendung bezüglich des Handrückens unterhalb des Befestigungselements, um die Drehachse drehbar, an dem Befestigungselement angeordnet ist; umfasst; wobei das erste Winkeleinstellelement und das zweite Winkeleinstellelement jeweils wenigstens eine Schwenkbegrenzung umfassen, welche die Extensions- und/oder Flexionsbewegung des ersten Schwenkhebels in ihrem Bewegungsumfang begrenzen.

Das erfindungsgemäße einstellbare Orthesengelenk ermöglicht es vorteilhaft, die Winkel der Extension und Flexion sowie der ulnaren und radialen Deviation der Hand je nach Gesundheitszustand und Therapiebedarf des Patienten zu kontrollieren. Der Winkel der Extension bzw. Flexion kann dabei vorteilhaft entweder bei 180° (γ₁ = γ₂ = 0°; ausgestreckte Hand) fixiert oder in verschiedenen Winkelbereichen von 180° bis 180° + γ₁ (Dorsalextension), von 180° bis 180° - γ₂ (Palmarflexion) sowie im gesamten Bewegungsbereich von 180° + γ₁ bis 180° - γ₂ mit γ₁, γ₂ > 0° kontrolliert freigegeben werden. Die Winkel γ₁, γ₂ lassen sich dazu vorteilhaft durch Drehung des oberen und/oder unteren Winkeleinstellelements um die Drehachse auf den jeweils gewünschten Winkel einstellen.

Dadurch kann eine erfindungsgemäße Orthese mit einem solchen einstellbaren Orthesengelenk den Heilungsverlauf vorteilhaft in der Weise begleiten, dass die Bewegungsfreiheit des Patienten kontrolliert erhöht werden kann, wobei aus therapeutischer Sicht zugelassene Bewegung kontrolliert geführt werden und gleichzeitig aus therapeutischer Sicht kritische Bewegungen vollständig oder teilweise eingeschränkt werden können. Auf diese Weise kann einer unnötigen Versteifung gesunder Gelenke der Hand vorteilhaft entgegengewirkt werden. Das erfindungsgemäße Orthesengelenk weist dabei eine besonders kompakte und robuste Bauweise auf, sodass eine damit ausgestattete erfindungsgemäße Orthese ebenfalls vorteilhaft kompakt ausgestaltet sein kann und den Patienten in seinem Alltag wenig behindert.

Weitere vorteilhafte Ausgestaltungen und Weiterbildungen, welche einzeln oder in Kombination miteinander einsetzbar sind, sind Gegenstand der abhängigen Ansprüche.

In einer Ausgestaltung der Erfindung hat es sich bewährt, wenn die wenigstens eine obere Schwenkbegrenzung an einer Unterseite des ersten, oberen Winkeleinstellelements angeordnet ist, wobei das erste, obere Winkeleinstellelement vorzugsweise scheibenförmig ausgebildet sein kann. Eine derart angeordnete obere Schwenkbegrenzung kann vorteilhaft zur Einstellung des Winkelbereichs von 180° bis 180° + γ₁, also zur Einstellung des Umfangs der Dorsalextension, verwendet werden.

In einer weiteren bevorzugten Ausgestaltung kann das erste, obere, vorzugsweise scheibenförmige, Winkeleinstellelement wenigstens zwei, bevorzugt vier, an der Unterseite angeordnete, obere Schwenkbegrenzungen umfassen, wobei die Schwenkbegrenzungen, um einen Winkel, welcher durch die jeweiligen Positionen der Schwenkbegrenzungen auf der Unterseite und den Schnittpunkt der Drehachse mit der Unterseite gebildet wird, zueinander beabstandet sind, und wobei die Schwenkbegrenzungen zueinander derart unterschiedlich ausgebildet sind, dass die Extensionsbewegung des ersten Schwenkhebels durch ein Zusammenwirken mit den verschiedenen Schwenkbegrenzungen in ihrem Bewegungsumfang unterschiedlich begrenzt wird. Zwei oder mehr, an der Unterseite angeordnete obere Schwenkbegrenzungen, welche zueinander unterschiedlich ausgebildet und in einem Winkel zueinander angeordnet sind, ermöglichen vorteilhaft die Einstellung verschiedener Winkelbereiche von 180° bis 180° + γ₁ für die Dorsalextension je nach dem speziellen Begrenzungswinkel γ₁, den die jeweils durch Drehung um die Drehachse ausgewählte Schwenkbegrenzung zulässt.

Darüber hinaus hat sich eine Ausgestaltung der Erfindung bewährt, bei der die wenigstens eine untere Schwenkbegrenzung an einer Oberseite des zweiten, unteren Winkeleinstellelements angeordnet ist, wobei das zweite, untere Winkeleinstellelement vorzugsweise scheibenförmig ausgebildet sein kann. Eine derart angeordnete untere Schwenkbegrenzung kann vorteilhaft zur Einstellung des Winkelbereichs von 180° bis 180° - γ₂, also zur Einstellung des Umfangs der Palmarflexion, verwendet werden.

Das zweite, untere, vorzugsweise scheibenförmige, Winkeleinstellelement kann in einer weiteren Ausgestaltung auch vorteilhaft wenigstens zwei, bevorzugt vier, an derer Oberseite angeordnete, untere Schwenkbegrenzungen umfassen, wobei die Schwenkbegrenzungen, um einen Winkel, welcher durch die jeweiligen Positionen der Schwenkbegrenzungen auf der Oberseite und den Schnittpunkt der Drehachse mit der Oberseite gebildet wird, zueinander beabstandet sind; und wobei die Schwenkbegrenzungen zueinander derart unterschiedlich ausgebildet sind, dass die Flexionsbewegung des ersten Schwenkhebels durch ein Zusammenwirken mit den verschiedenen Schwenkbegrenzungen in ihrem Bewegungsumfang unterschiedlich begrenzt wird. Zwei oder mehr, an der Oberseite angeordnete untere Schwenkbegrenzungen, welche zueinander unterschiedlich ausgebildet und in einem Winkel zueinander angeordnet sind, ermöglichen vorteilhaft die Einstellung verschiedener Winkelbereiche von 180° bis 180° - γ₂ für die Palmarflexion je nach dem speziellen Begrenzungswinkel γ₂, den die jeweils durch Drehung um die Drehachse ausgewählte Schwenkbegrenzung zulässt.

Besonders bevorzugt können die Schwenkbegrenzungen als Aussparungen im ersten und/oder zweiten Winkeleinstellelement ausgebildet sein, sodass beim ersten Winkeleinstellelement eine Dicke des Winkeleinstellelements von seiner Unterseite in Richtung seiner Oberseite derart verringert ist, dass der erste Schwenkhebel bei einer Schwenkbewegung zum ersten, oberen Winkeleinstellelement hin, nach einem überstrichenen Winkel γ₁ ≥ 0° blockiert wird; und beim zweiten Winkeleinstellelement eine Dicke des Winkeleinstellelements von seiner Oberseite in Richtung seiner Unterseite derart verringert ist, dass der erste Schwenkhebel bei einer Schwenkbewegung zum zweiten, unteren Winkeleinstellelement hin, nach einem überstrichenen Winkel γ₂ ≥ 0° blockiert wird. Als Aussparungen in den jeweiligen Winkeleinstellelementen ausgestaltete Schwenkbegrenzungen stellen eine besonders kompakte und dabei robuste Lösung zur Winkelbegrenzung der Schwenkbewegung des ersten Schwenkhebels dar, wobei vorteilhaft zusätzliche Bauteile an den jeweiligen Winkeleinstellelementen eingespart werden können.

In einer weiteren bevorzugten Ausgestaltung der Erfindung, kann an der Unterseite des ersten, oberen Winkeleinstellelements wenigstens ein oberes Einrastmittel angeordnet sein, welches eingerichtet ist, das obere Winkeleinstellelement mit dem Befestigungselement über wenigstens ein, an einer Oberseite des Befestigungselements angeordnetes und korrespondierend zum oberen Einrastmittel ausgebildetes Einrastloch, reversibel zu verbinden, sodass eine unbeabsichtigte Drehung des ersten Winkeleinstellelements um die Drehachse verhindert wird.

Alternativ oder kumulativ dazu, kann in einer Ausgestaltung der Erfindung an der Oberseite des zweiten, unteren Winkeleinstellelements wenigstens ein unteres Einrastmittel angeordnet ist, welches eingerichtet ist, das untere Winkeleinstellelement mit dem Befestigungselement über wenigstens ein, an einer Unterseite des Befestigungselements angeordnetes und korrespondierend zum unteren Einrastmittel ausgebildetes Einrastloch, reversibel zu verbinden, sodass eine unbeabsichtigte Drehung des zweiten Winkeleinstellelements um die Drehachse verhindert wird.

Derartige untere und/oder obere Einrastmittel mit entsprechend korrespondierend ausgebildeten Einrastlöchern am Befestigungselement, ermöglichen vorteilhaft eine reversible Fixierung des jeweiligen Winkeleinstellelements, vorzugsweise unabhängig voneinander, am Befestigungselement in einer Position, bei der die jeweils ausgewählte obere und/oder untere Schwenkbegrenzung sauber in Wirkverbindung mit dem ersten Schwenkhebel kommen kann, wenn eine Schwenkbewegung ausgeführt wird. Ein unbeabsichtigtes Verdrehen des jeweiligen Winkeleinstellelements um die Drehachse, was bei einer ausgeführten Schwenkbewegung nachteilig zu einer unsauberen Wirkverbindung führen könnte, wird dadurch vorteilhaft verhindert. Soll jedoch, insbesondere bei zwei oder mehr, an den jeweiligen Winkeleinstellelementen angeordneten Schwenkbegrenzungen, eine jeweils andere Schwenkbegrenzung ausgewählt werden, kann durch relativ geringen, jedoch bewussten Krafteinsatz das Einrasten des jeweiligen Einrastmittels überwunden und eine andere Schwenkbegrenzung in eine Wirkposition gebracht werden.

Darüber hinaus hat sich eine Ausgestaltung der vorliegenden Erfindung bewährt, bei der am ersten Schwenkhebel wenigstens ein erster Anschlag und wenigstens ein zweiter Anschlag angeordnet sind, welche eingerichtet sind, einen Winkel der Schwenkbewegung des zweiten Schwenkhebels zu begrenzen, wobei die Anschläge vorzugsweise reversibel mit dem ersten Schwenkhebel verbindbar ausgebildet sind. Derartige, am ersten Schwenkhebel angeordnete Anschläge ermöglichen vorteilhaft die Begrenzung auch der Schwenkbewegung des zweiten Schwenkhebels um dessen Drehgelenk zur Ausführung einer ulnaren und radialen Deviation der Hand.

Schließlich hat sich eine Ausgestaltung der Erfindung bewährt, bei der der zweite Schwenkhebel wenigstens einen Schlitten umfasst, welcher eingerichtet ist, den zweiten Schwenkhebel mit einem Verbindungselement zu einer Hand bewegbar zu verbinden, wobei das Verbindungselement dazu vorzugsweise eine Schiene umfasst, welche korrespondierend zu besagtem Schlitten ausgebildet ist. Ein derartiger, bewegbar mit einem Verbindungselement zu einer Hand verbundener, zweiter Schwenkhebel ermöglicht es vorteilhaft, insbesondere bei einer kombinierten Bewegung der Hand, beispielsweise bei einer Palmarflexion bei gleichzeitiger ulnarer oder radialer Deviation, den sich ändernden geometrischen Verhältnissen (beispielsweise einer Veränderungen der optimalen Längenverhältnisse bezüglich des Abstand des Drehgelenks des zweiten Schwenkhebels zu einem Verbindungspunkt zwischen zweitem Schwenkhebel und Verbindungselement) Rechnung zu tragen.

Die vorliegende Erfindung betrifft darüber hinaus auch eine Orthese zur kontrollierten Bewegung und/oder Fixierung einer Hand oder einer Hand und eines Unterarms eines Patienten, mit einem einstellbaren Orthesengelenk wie zuvor beschrieben.

Diese sowie zusätzliche Einzelheiten und weitere Vorteile der Erfindung werden nachfolgend an Hand bevorzugter Ausführungsbeispiele, auf welche die vorliegende Erfindung jedoch nicht beschränkt ist, und in Verbindung mit der beigefügten Zeichnung beschrieben.

Darin zeigen schematisch:
- Fig. 1: eine Ausgestaltung eines erfindungsgemäßen Orthesengelenks in einer perspektivischen Explosions-Seitenansicht;
- Fig. 2: die Ausgestaltung aus Fig. 1 in einer perspektivischen Explosions-Draufsicht;
- Fig. 3: in den Teilfiguren a bis d Seitenansichten von Ausschnitten einer weiteren Ausgestaltung eines erfindungsgemäßen Orthesengelenks, welche verschiedene, durch die jeweils ausgewählten Schwenkbegrenzungen am jeweiligen Winkeleinstellelement vorgegebene Bewegungswinkelbereiche exemplarisch darstellen;
- Fig. 4: in den Teilzeichnung a bis c eine Ausgestaltung eines ersten, oberen Winkeleinstellelements mit Blick auf dessen Unterseite (Fig. 4a); eine Ausgestaltung eines Befestigungselements mit Blick auf dessen Oberseite (Fig. 4b) und ein zweites, unteres Winkeleinstellelement mit Blick auf dessen Oberseite (Fig. 4c);
- Fig. 5: in einer Draufsicht eine weitere Ausgestaltung eines erfindungsgemäßen Orthesengelenks mit einem, über einen Schlitten mit einem Verbindungselement zu einer Hand, bewegbar verbundenen zweiten Schenkhebel; und
- Fig. 6: in einer Draufsicht eine Ausgestaltung einer erfindungsgemäßen Orthese bei Benutzung.

Bei der nachfolgenden Beschreibung bevorzugter Ausführungsformen der vorliegenden Erfindung bezeichnen gleiche Bezugszeichen gleiche oder vergleichbare Komponenten.

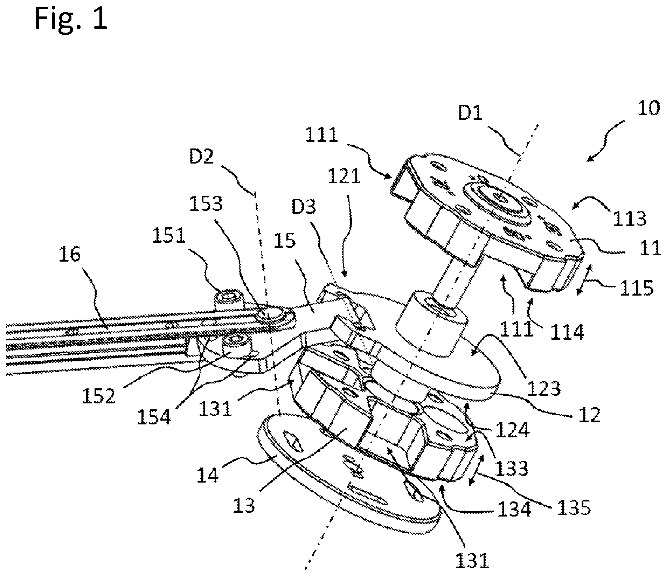

In **Fig. 1** ist eine Ausgestaltung eines erfindungsgemäßen Orthesengelenks 10 in einer perspektivischen Explosions-Seitenansicht gezeigt.

Ein erfindungsgemäßes einstellbares Orthesengelenk 10 zur kontrollierten Bewegung und/oder Fixierung einer Hand eines Patienten umfasst wenigstens einen ersten Schwenkhebel 15 zur Ausführung einer Extensions- und/oder Flexionsbewegung der Hand und einen, am ersten Schwenkhebel 15 schwenkbar angeordneten, zweiten Schwenkhebel 16 zur Ausführung einer ulnaren und radialen Deviation der Hand. Das Orthesengelenk 10 umfasst dazu ein, vorzugsweise scheibenförmiges, Befestigungselement 12 an dem der erste Schwenkhebel 15, vorzugsweise über ein Schwenkgelenk 121, befestigt und um eine Drehachse D3 schwenkbar ausgestaltet ist. Der zweite Schwenkhebel 16 kann insbesondere über ein Drehgelenk 153 mit dem ersten Schwenkhebel 15 wirkverbunden sein und sich dadurch um eine Drehachse D2, welche vorzugsweise senkrecht zum ersten Schwenkhebel 15 ausgerichtet ist, schwenken lassen.

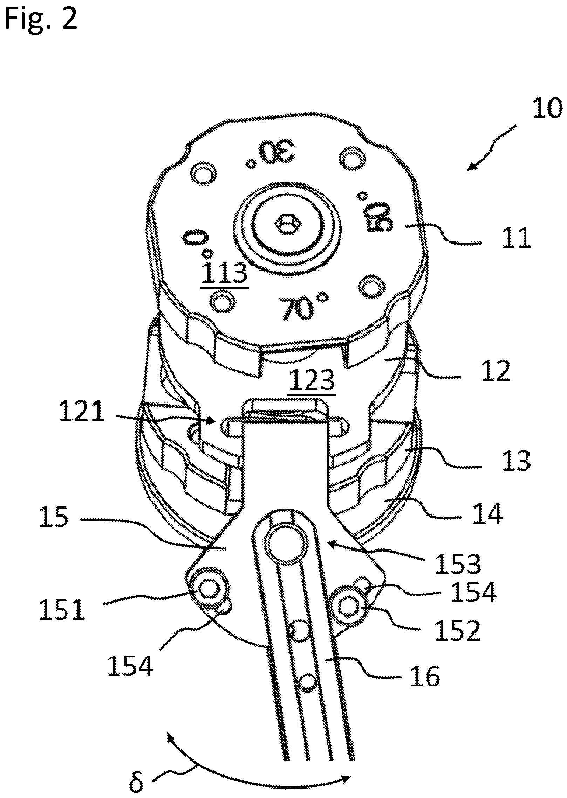

**Fig. 2** zeigt die Ausgestaltung aus Fig. 1 in einer perspektivischen Explosions-Draufsicht.

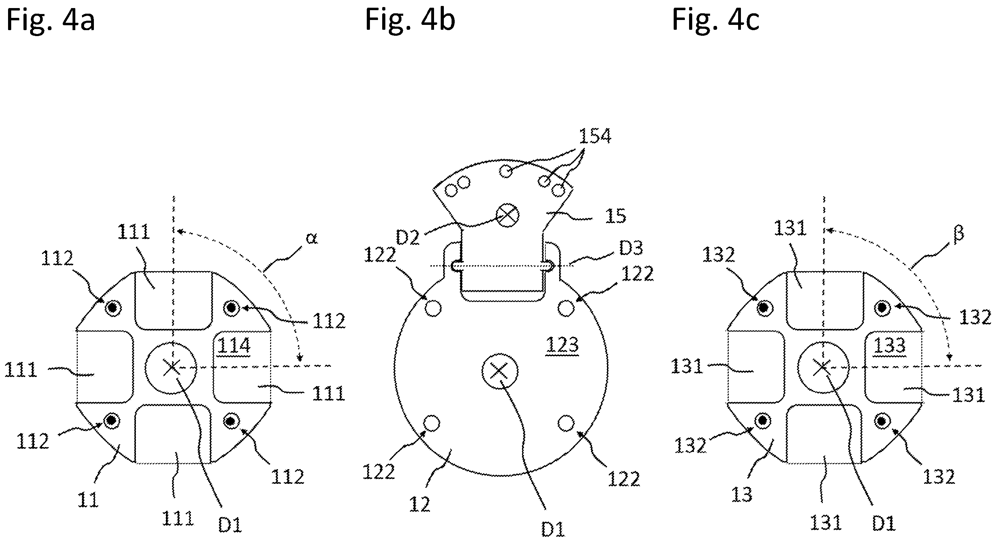

Wie hier zu sehen ist, kann der zweite Schwenkhebel 16 dabei vorzugsweise einen Schwenkwinkel δ überstreichen, wobei der Schwenkwinkel δ je nach Auslenkung des ersten Schwenkhebels 15 jeweils auf unterschiedlichen Ebenen eines Ebenenbüschels liegt, dessen Trägergerade gerade die Drehachse D3 des ersten Schwenkhebels 15 ist. In Fig. 2 ist auch dargestellt, dass am ersten Schwenkhebel 15 wenigstens ein erster Anschlag 151 und wenigstens ein zweiter Anschlag 152 angeordnet sein können, welche eingerichtet sind, den Winkel δ der Schwenkbewegung des zweiten Schwenkhebels 16 zu begrenzen, wobei die Anschläge 151 und 152 vorzugsweise reversibel mit dem ersten Schwenkhebel 15 verbindbar ausgebildet sind. Die Anschläge 151 und 152 können insbesondere über Fixierelemente 154 mit dem ersten Schwenkhebel 15 reversibel verbindbar sein. In den Fig. 1 und Fig. 2 sind dazu Fixierelemente 154 angedeutet, welche als einfache Löcher ausgestaltet sind und durch die Anschläge 151 und 152 einfach gesteckt und verschraubt werden können. Besagte, als Löcher ausgestaltete Fixierelement 154 können dabei langezogen, wie in Fig. 1 und Fig. 2 angedeutet, es können allerdings auch mehrere, dicht nebeneinander platzierte Löcher sein, wie in Fig. 4b gezeigt. Als Fixierelemente 154 können allerdings auch insbesondere magnetische Halteplätze auf dem ersten Schwenkhebel 15 angeordnet sein, auf denen die Anschläge 151 und 152 reversibel platziert werden können.

Das erfindungsgemäße Orthesengelenk 10 umfasst ferner ein erstes, oberes, vorzugsweise scheibenförmiges, Winkeleinstellelement 11, welches bei Verwendung bezüglich eines Handrückens oberhalb des Befestigungselements 12, um eine Drehachse D1 drehbar, an dem Befestigungselement 12 angeordnet ist und ein zweites, unteres, vorzugsweise scheibenförmiges, Winkeleinstellelement 13, welches bei Verwendung bezüglich des Handrückens unterhalb des Befestigungselements 12, um die Drehachse D1 drehbar, an dem Befestigungselement 12 angeordnet ist. Das erste Winkeleinstellelement 11 und das zweite Winkeleinstellelement 13 umfassen dabei jeweils wenigstens eine Schwenkbegrenzung 111 bzw. 131, welche die Extensions- und/oder Flexionsbewegung des ersten Schwenkhebels 15 in ihrem Bewegungsumfang begrenzen. Die wenigstens eine obere Schwenkbegrenzung 111 kann dabei insbesondere an einer Unterseite 114 des ersten, oberen, vorzugsweise scheibenförmigen, Winkeleinstellelements 11 angeordnet sein und die wenigstens eine untere Schwenkbegrenzung 131 kann insbesondere an einer Oberseite 133 des zweiten, unteren, vorzugsweise scheibenförmigen, Winkeleinstellelements 13 angeordnet sein. Im vorliegenden Beispiel sind sowohl das erste, obere Winkeleinstellelement 11, das zweite, untere Winkeleinstellelement 13 als auch das Befestigungselement 12 als näherungsweise runde Scheiben dargestellt, welche zueinander einen ähnlichen Durchmesser aufweisen und sandwichartig übereinander angeordnet sind, die einzelnen Ebenen sind mit anderen Worten parallel zueinander angeordnet. Unter dem Begriff "scheibenförmig" sollen im Zusammenhang der vorliegenden Erfindung jedoch auch Bauteile mit von einem Kreis abweichenden Umrissen verstanden werden, insbesondere können das erste, obere Winkeleinstellelement 11, das zweite, untere Winkeleinstellelement 13 und/oder das Befestigungselement auch jeweils einen polygonalen Umriss aufweisen, wie auch bereits in Fig. 2 angedeutet. Darüber hinaus kann das erste, obere Winkeleinstellelement 11 auch als ein scheibenförmiges Bauteil mit näherungsweise halbkreisförmigem Umriss ausgestaltet sein, welches senkrecht zur Ebene des Befestigungsmittels 12, an diesem angeordnet ist (hier nicht dargestellt). Die wenigstens eine obere Schwenkbegrenzung 111 kann in diesem Fall vorzugsweise als Anschlag ausgestaltet sind, welcher aus der Ebene des senkrecht angeordneten ersten, oberen Winkeleinstellelements 11 herausragt und dadurch insbesondere die dorsale Extensionsbewegung des ersten Schwenkhebels 15 in ihrem Bewegungsumfang begrenzen kann.

Bevorzugt ist allerdings eine Ausgestaltung eines erfindungsgemäßen Orthesengelenks 10 mit wenigstens zwei, besonders bevorzugt - wie in den Fig. 1 und 2 dargestellt - mit vier, an der Unterseite 114 des ersten, oberen, vorzugsweise scheibenförmigen, Winkeleinstellelements 11 angeordneten, oberen Schwenkbegrenzungen 111 bzw. mit wenigstens zwei, besonders bevorzugt mit vier, an der Oberseite 133 des zweiten, unteren, vorzugsweise scheibenförmigen, Winkeleinstellelements 13 angeordneten, unteren Schwenkbegrenzungen 131. Die Anzahl der an einem Winkeleinstellelement 11 oder 13 angeordneten Schwenkbegrenzungen 111 oder 131 ist allerdings nicht auf die Zahlen zwei oder vier begrenzt, sondern kann variieren und auch eine ungerade Anzahl annehmen. Die maximale Anzahl hängt im Prinzip nur vom Verhältnis der gewählten Breite des ersten Schwenkhebels 15 im Verhältnis zum Radius des jeweiligen Winkeleinstellelements 11 oder 13 ab. Je schmäler der erste Schwenkhebel 15 ist, desto mehr korrespondierend dazu ausgebildete Schwenkbegrenzungen 111 bzw. 131 können bei gleichem Radius der jeweiligen Winkeleinstellelementen 11 bzw. 13 an besagten Winkeleinstellelementen 11 oder 13 angeordnet werden.

Sind mehr als jeweils eine obere Schwenkbegrenzung 111 und untere Schwenkbegrenzung 131 and den Winkeleinstellelementen 11 bzw. 13 angeordnet, können die oberen Schwenkbegrenzungen 111, um einen Winkel α, welcher durch die jeweiligen Positionen der Schwenkbegrenzungen 111 auf der Unterseite 114 und den Schnittpunkt der Drehachse D1 mit der Unterseite 114 gebildet wird, zueinander beabstandet sein. Entsprechend können die unteren Schwenkbegrenzungen 131, um einen Winkel β, welcher durch die jeweiligen Positionen der Schwenkbegrenzungen 131 auf der Oberseite 133 und den Schnittpunkt der Drehachse D1 mit der Oberseite 133 gebildet wird, zueinander beabstandet sein (vgl. dazu auch Fig. 4). Im hier gezeigten Beispiel mit jeweils vier Schwenkbegrenzungen 111 und 131 pro Winkeleinstellelement 11 und 13 betragen die Winkel α und β jeweils 90°. Bei geeignet schmalem ersten Schwenkhebel 15 kann sich die Anzahl der Schwenkbegrenzungen 111 und 131 am ersten, oberen Winkeleinstellelement 11 und am zweiten, unteren Winkeleinstellelement 13 auch voneinander unterscheiden und die Winkel α und β können entsprechend ungleich sein.

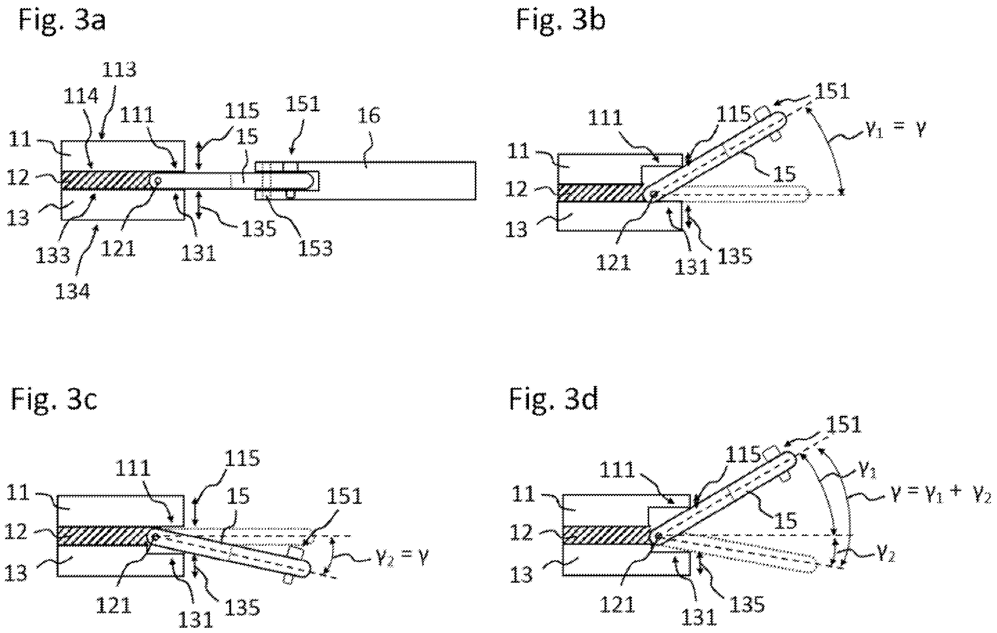

**Fig. 3** zeigt in den Teilfiguren **a** bis **d** Seitenansichten von Ausschnitten einer weiteren Ausgestaltung eines erfindungsgemäßen Orthesengelenks 10, welche verschiedene, durch die jeweils ausgewählten Schwenkbegrenzungen 111 und 131 am jeweiligen Winkeleinstellelement 11 und 13 vorgegebene Bewegungswinkelbereiche exemplarisch darstellen.

Wie in Figuren 3a bis d gezeigt, können die oberen Schwenkbegrenzungen 111 bzw. die unteren Schwenkbegrenzungen 131 bevorzugt zueinander derart unterschiedlich ausgebildet sein, dass die Extensions- bzw. Flexionsbewegung des ersten Schwenkhebels 15 durch ein Zusammenwirken mit den verschiedenen oberen 111 bzw. unteren 131 Schwenkbegrenzungen in ihrem Bewegungsumfang unterschiedlich begrenzt wird. Die Schwenkbegrenzungen 111 bzw. 131 können dazu besonders bevorzugt als Aussparungen im ersten 11 und/oder zweiten 13 Winkeleinstellelement ausgebildet sein, sodass beim ersten Winkeleinstellelement 11 eine Dicke 115 des Winkeleinstellelements 11 von seiner Unterseite 114 in Richtung seiner Oberseite 113 derart verringert ist, dass der erste Schwenkhebel 15 bei einer Schwenkbewegung zum ersten, oberen Winkeleinstellelement 11 hin, nach einem überstrichenen Winkel γ₁ ≥ 0° blockiert wird; und beim zweiten Winkeleinstellelement 13 eine Dicke 135 des Winkeleinstellelements 13 von seiner Oberseite 133 in Richtung seiner Unterseite 134 derart verringert ist, dass der erste Schwenkhebel 15 bei einer Schwenkbewegung zum zweiten, unteren Winkeleinstellelement 13 hin, nach einem überstrichenen Winkel γ₂ ≥ 0° blockiert wird.

**Fig. 3a** zeigt beispielsweise eine Einstellung des erfindungsgemäßen Orthesengelenks 10, bei der die obere 111 sowie die untere 131 Schwenkbegrenzung gleichartig ausgebildet sind und die Dicke 115 des ersten, oberen Winkeleinstellelements 11 bzw. die Dicke 135 des zweiten, unteren Winkeleinstellelements 13 nicht verringern. Die durch den ersten Schwenkhebel 15 überstrichenen Winkel γ₁ und γ₂ sind in diesem Fall gleich 0° - die Extensions- bzw. Flexionsbewegung einer Hand, welche durch eine Orthese mit dem erfindungsgemäßen Orthesengelenk 10 gestützt ist, wäre fixiert. Die Hand wäre gestreckt und könnte lediglich eine, über den zweiten Schwenkhebel 16 geführte, ulnare und radiale Deviation ausführen. In den im Folgenden beschriebenen **Fig. 3b** bis **d** ist der zweite Schwenkhebel 16 der Übersichtlichkeit wegen nicht gezeichnet, die Funktionsweise des zweiten Schenkhebels 16 bleibt jedoch wie zuvor beschrieben. **Fig. 3b** zeigt nun eine Einstellung des erfindungsgemäßen Orthesengelenks 10, bei der die obere Schwenkbegrenzung 111 als Aussparung ausgestaltet ist, wobei die Dicke 115 des Winkeleinstellelements 11 von seiner Unterseite 114 in Richtung seiner Oberseite 113 derart verringert ist, dass der erste Schwenkhebel 15 bei einer Schwenkbewegung zum ersten, oberen Winkeleinstellelement 11 hin, nach einem überstrichenen Winkel γ₁ > 0° blockiert wird. Die untere Schwenkbegrenzung 131 verringert die Dicke 135 des zweiten, unteren Winkeleinstellelements 13 nicht, so dass der durch den ersten Schwenkhebel 15 überstreichbare Winkel γ₂, wie in Fig. 3a, gleich 0° ist, da die untere Schwenkbegrenzung 131 jegliche Schwenkbewegung des ersten Schwenkhebels 15 blockiert. Eine derartige Einstellung des erfindungsgemäßen Orthesengelenks 10, welche durch einfache Drehung des oberen Winkeleinstellelements 11 um die Drehachse D1 bis hin zu einer Position des ersten, oberen Winkeleinstellelements 11, bei der die für den gewünschten Winkel γ₁ geeignet ausgestaltete Schwenkbegrenzung 111 in Wirkverbindung mit dem ersten Schwenkhebel 15 treten kann, erreichbar ist, ermöglicht beispielsweise ausschließlich eine Dorsalextension der jeweiligen Hand, wobei in Kombination dazu wieder eine ulnare und radiale Deviation ausführbar sein kann. Fig. 3c zeigt entsprechend exemplarisch eine Einstellung eines erfindungsgemäßen Orthesengelenks 10, bei dem das erste, obere Winkeleinstellelement 11, wie in Fig. 3a, jegliche Schwenkbewegung des ersten Schwenkhebels 15 blockiert, während das zweite, untere Winkeleinstellelement 13 wiederum durch Drehung um die Drehachse D1 in eine Position gebracht ist, in der die untere, mit dem ersten Schwenkhebel 15 bei einer Bewegung in Wechselwirkung tretende, Schwenkbegrenzung 131 als Aussparungen im zweiten Winkeleinstellelement 13 ausgebildet ist und die Dicke 135 des Winkeleinstellelements 13 von seiner Oberseite 133 in Richtung seiner Unterseite 134 derart verringert ist, dass der erste Schwenkhebel 15 bei einer Schwenkbewegung zum zweiten, unteren Winkeleinstellelement 13 hin, nach einem überstrichenen Winkel γ₂ ≥ 0° blockiert wird. Im vorliegenden Beispiel ist der Betrag des überstrichenen Winkels γ₂ kleiner eingezeichnet als der des Winkels γ₁ in Fig. 3b. Besagte Beträge können aber auch gleich sein, je nachdem, welche Schwenkbegrenzungen 111 bzw. 131 vom Benutzer gewählt werden. In Fig. 1 und Fig. 2 sind dazu auf der Oberseite 113 des ersten Winkeleinstellelements 11 beispielsweise oberhalb jeder der hier eigenzeichneten vier oberen Schwenkbegrenzungen 111 die entsprechenden maximalen Bewegungswinkel in Grad angegeben - hier 0°, 30°, 50° und 70° - es sind aber natürlich auch andere Winkelvorgaben möglich. Eine entsprechende Markierung kann auch vorzugsweise am zweiten, unteren Winkeleinstellelement 13 angebracht sein (hier nicht eingezeichnet).

**Fig. 3d** zeigt schließlich eine Einstellung des Orthesengelenks 10, bei der beide Schwenkbegrenzungen 111 und 131 Schwenkwinkel γ₁ und γ₂ größer 0° zulassen. Der mögliche Gesamtbewegungswinkel γ des ersten Schwenkhebels 15 ergibt sich in diesem Fall aus der Summe der beiden Winkel γ₁ und γ₂. Im gezeigten Beispiel sind besagte Winkel γ₁ und γ₂ ungleich, so dass sich eine unsymmetrische, maximal mögliche Gesamtbewegung ergibt; die beiden Winkel γ₁ und γ₂ können aber auch gleich sein (nicht dargestellt), was zu einer symmetrischen, maximal möglichen Gesamtbewegung führen würde.

In **Fig. 4** ist in den Teilzeichnung **a** bis **c** eine Ausgestaltung eines ersten, oberen Winkeleinstellelements 11 mit Blick auf dessen Unterseite 114 (**Fig. 4a**); eine Ausgestaltung eines Befestigungselements 12 mit Blick auf dessen Oberseite 123 (**Fig. 4b**) und ein zweites, unteres Winkeleinstellelement 13 mit Blick auf dessen Oberseite 133 (**Fig. 4c**) gezeigt.

An der Unterseite 114 des ersten, oberen Winkeleinstellelements 11 kann vorzugsweise wenigstens ein oberes Einrastmittel 112 angeordnet sein, welches eingerichtet ist, das obere Winkeleinstellelement 11 mit dem Befestigungselement 12 über wenigstens ein, an einer Oberseite 123 des Befestigungselements 12 angeordnetes und korrespondierend zum oberen Einrastmittel 112 ausgebildetes Einrastloch 122, reversibel zu verbinden, sodass eine unbeabsichtigte Drehung des ersten Winkeleinstellelements 11 um die Drehachse D1 verhindert wird. Entsprechend kann, wie gezeigt, an der Oberseite 133 des zweiten, unteren Winkeleinstellelements 13 wenigstens ein unteres Einrastmittel 132 angeordnet sein, welches eingerichtet ist, das untere Winkeleinstellelement 13 mit dem Befestigungselement 12 über wenigstens ein, an einer Unterseite 124 des Befestigungselements 12 angeordnetes und korrespondierend zum unteren Einrastmittel 132 ausgebildetes Einrastloch 122 (hier nicht gezeigt), reversibel zu verbinden, sodass eine unbeabsichtigte Drehung des zweiten Winkeleinstellelements 13 um die Drehachse D1 verhindert wird.

Die Einrastmittel 112 und 132 - in den Fig. 4a und 4c sind exemplarisch jeweils vier am oberen 11 bzw. unteren 13 Winkeleinstellelement gezeigt - können insbesondere als kleine, hohle Madenschrauben ausgebildet sein, wobei sich im jeweiligen Hohlraum einer jeden Madenschraube eine Feder befinden kann, welche am offenen Ende der Madenschraube eine Kugel nach außen drückt. Die jeweilige Kugeloberfläche rastet dann in das dafür vorgesehene, vorzugsweise als Nut ausgebildete, Einrastloch 122 am Befestigungselement 12 ein. Einrastlöcher 122, insbesondere als Nut ausgebildete Einrastlöcher 122, können dazu, wie bereits erwähnt, sowohl an der Oberseite 123 als auch an der Unterseite 124 des Befestigungselements 12 angeordnet sein. Alternativ oder kumulativ dazu können das obere 112 und/oder das untere 132 Einrastmittel sowie die entsprechenden Einrastlöcher 122 auch als einfache Ausfräsungen (Nut plus Vertiefung, Verzahnung, etc.) ausgestaltet sein. Vorteilhaft bei der Verwendung von Madenschrauben, wie zuvor beschrieben, ist, dass dadurch der Charakter des Einklickens bzw. Einrastens genau definiert werden kann und des sich gleichzeitig um eine elegante und dabei kostengünstige Lösung handelt.

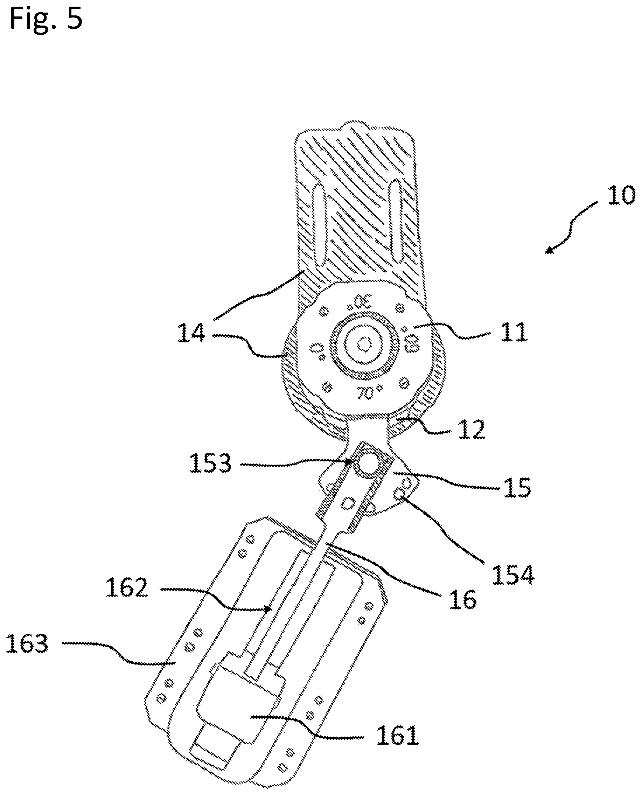

**Fig. 5** zeigt in einer Draufsicht eine weitere Ausgestaltung eines erfindungsgemäßen Orthesengelenks 10 mit einem, über einen Schlitten 161 mit einem Verbindungselement 163 zu einer Hand, bewegbar verbundenen zweiten Schenkhebel 16.

Zur Befestigung an einer Orthese 1 zur kontrollierten Bewegung und/oder Fixierung einer Hand oder einer Hand und eines Unterarms eines Patienten, kann das erfindungsgemäße Orthesengelenk 10 nach unten, also zum Handrücken bzw. zum Unterarm hin, durch eine Deckplatte 14 abgeschlossen werden, wie in den Fig. 1, 2, 5 und 6 gezeigt. Besagte Deckplatte 14 kann vorzugsweise aus einem beliebigen Kunststoff gebildet sein; das erste, obere 11 und zweite, untere 13 Winkeleinstellelement sowie das Befestigungselement 12 können insbesondere aus Aluminium, Edelstahl oder einem medizinischen Kunststoff gebildet sein.

Fig. 5 zeigt auch, dass die Deckplatte 14 eine längliche Form aufweisen kann, welche sich, wenigstens in eine Richtung jenseits der Ausdehnung des ersten, oberen 11 und/oder des zweiten, unteren 13 Winkeleinstellelements, beziehungsweise jenseits des Befestigungselements 12 hin erstrecken kann. Eine derartige länglich Form ermöglicht vorteilhaft eine mittelbare oder unmittelbare Verbindung des Orthesengelenks 10 über eine solche Deckplatte 14 mit dem Unterarm eines Patienten.

Der zweite Schwenkhebel 16 kann zudem wenigstens einen Schlitten 161 umfassen, welcher eingerichtet ist, den zweiten Schwenkhebel 16 mit einem Verbindungselement 163 zu einer Hand bewegbar zu verbinden, wobei das Verbindungselement 163 dazu vorzugsweise eine Schiene 162 umfassen kann, welche korrespondierend zu besagtem Schlitten 161 ausgebildet ist. In Fig. 5 ist besagtes Verbindungselement 163 als Kunststoffbauteil dargestellt. Es kann jedoch auch wiederum aus Aluminium oder Edelstahl gefertigt sein. Über den Schlitten 161 ist der zweite Schenkhebel 16 in dieser Ausgestaltung vorteilhaft bewegbar mit dem Verbindungselement 163 wirkverbunden, wobei der Schlitten 161, wie hier gezeigt, vorzugsweise in einer korrespondierend ausgestalteten Schiene 162 - hier beispielsweise als Aussparung im Verbindungselement 163 ausgebildet - laufen kann. Bei der Ausführung einer kombinierten Bewegung der Hand, zum Beispiel eine Palmarflexion bei gleichzeitiger Deviation oder bei einer Dorsalextension bei gleichzeitiger Deviation, kann sich das Verbindungsmittel 163 dadurch vorteilhaft gegenüber dem zweiten Schenkhebel 16 verschieben und sich den sich bei einer Bewegung ändernden geometrischen Verhältnissen anpassen.

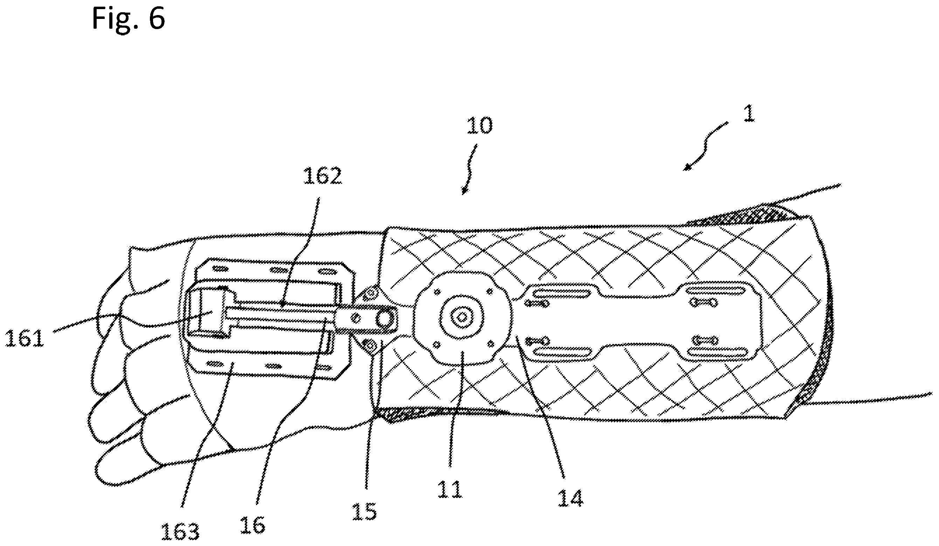

**Fig. 6** zeigt schließlich in einer Draufsicht eine Ausgestaltung einer erfindungsgemäßen Orthese 1 bei Benutzung.

Die vorliegende Erfindung betrifft eine einstellbares Orthesengelenk 10 zur kontrollierten Bewegung und/oder Fixierung einer Hand eines Patienten sowie eine Orthese 1 mit einem derartigen Orthesengelenk 10, welches sich dadurch auszeichnet, dass es wenigstens ein Befestigungselement 12 zur schwenkbaren Befestigung eines ersten Schwenkhebels 15; wenigstens ein erstes, oberes Winkeleinstellelement 11, und wenigstens ein zweites, unteres Winkeleinstellelement 13 umfasst; wobei das erste Winkeleinstellelement 11 und das zweite Winkeleinstellelement 13 jeweils wenigstens eine Schwenkbegrenzung 111 bzw. 131 umfassen, welche die Extensions- und/oder Flexionsbewegung eines ersten Schwenkhebels 15 in ihrem Bewegungsumfang begrenzen. Das erfindungsgemäße einstellbare Orthesengelenk 10 ermöglicht vorteilhaft, die Winkel der Extension und Flexion sowie der ulnaren und radialen Deviation der Hand je nach Gesundheitszustand und Therapiebedarf des Patienten zu kontrollieren und ist dabei vorteilhaft kompakt und robust.

### Bezugszeichenliste

- 1: Orthese
- 10: Orthesengelenk
- 11: Erstes, oberes Winkeleinstellelement
111 Obere Schwenkbegrenzung
112 Oberes Einrastmittel
113 Oberseite des ersten Winkeleinstellelements (11)
114 Unterseite des ersten Winkeleinstellelements (11)
115 Dicke des ersten, oberen Winkeleinstellelements (11)
- 12: Befestigungselement
121 Schwenkgelenk für ersten Schwenkhebel (15)
122 Einrastloch
123 Oberseite des Befestigungselements (12)
124 Unterseite des Befestigungselements (12)
- 13: Zweites, unteres Winkeleinstellelement
131 Untere Schwenkbegrenzung
132 Unteres Einrastmittel
133 Oberseite des zweiten Winkeleinstellelements (13)
134 Unterseite des zweiten Winkeleinstellelements (13)
135 Dicke des zweiten, unteren Winkeleinstellelements (13)
- 14: Deckplatte
- 15: Erster Schwenkhebel für Extension/Flexion (E/F)
151 Erster Anschlag
152 Zweiter Anschlag
153 Drehgelenk für den zweiten Schwenkhebel (16)
154 Fixierelement für Anschläge (151; 152)
- 16: Zweiter Schwenkhebel für ulnare/radiale Deviation (urD) der Hand
161 Schlitten
162 Schiene
163 Verbindungselement zur Hand
- α: Winkel zwischen den oberen Schwenkbegrenzungen (111)
- β: Winkel zwischen den unteren Schwenkbegrenzungen (131)
- γ: Gesamtbewegungswinkel des ersten Schwenkhebels (15); γ = γ₁ + γ₂
- γ₁: Winkel der Schwenkbewegung des ersten Schwenkhebels (15) zum ersten, oberen Winkeleinstellelement (11) hin
- γ₂: Winkel der Schwenkbewegung des ersten Schwenkhebels (15) zum zweiten, unteren Winkeleinstellelement (13) hin
- δ: Winkel der Schwenkbewegung des zweiten Schwenkhebels (16)
- D1: Drehachse der Winkeleinstellelemente (11; 13)
- D2: Drehachse des Drehgelenks (153) für den zweiten Schwenkhebel (16)
- D3: Drehachse des Schwenkgelenks (121) für den ersten Schwenkhebel (15)

## Patentansprüche

1. Einstellbares Orthesengelenk (10) zur kontrollierten Bewegung und/oder Fixierung einer Hand eines Patienten, wenigstens umfassend
- einen ersten Schwenkhebel (15) zur Ausführung einer Extensions- und/oder Flexionsbewegung der Hand;
- einen, am ersten Schwenkhebel (15) schwenkbar angeordneten, zweiten Schwenkhebel (16) zur Ausführung einer ulnaren und/oder radialen Deviation der Hand;
wobei
- das Orthesengelenk (10)
- ein Befestigungselement (12) zur schwenkbaren Befestigung des ersten Schwenkhebels (15) umfasst ;
**dadurch gekennzeichnet, dass**
das Orthesengelenk
- ein erstes, oberes Winkeleinstellelement (11), welches bei Verwendung bezüglich eines Handrückens oberhalb des Befestigungselements (12), um eine Drehachse (D1) drehbar, an dem Befestigungselement (12) angeordnet ist; und
- ein zweites, unteres Winkeleinstellelement (13), welches bei Verwendung bezüglich des Handrückens unterhalb des Befestigungselements (12), um die Drehachse (D1) drehbar, an dem Befestigungselement (12) angeordnet ist;
umfasst;
- wobei das erste Winkeleinstellelement (11) und das zweite Winkeleinstellelement (13) jeweils wenigstens eine Schwenkbegrenzung (111; 131) umfassen, welche die Extensions- und/oder Flexionsbewegung des ersten Schwenkhebels (15) in ihrem Bewegungsumfang begrenzen.

2. Orthesengelenk (10) nach Anspruch 1, **dadurch gekennzeichnet, dass** die wenigstens eine obere Schwenkbegrenzung (111) an einer Unterseite (114) des ersten, oberen Winkeleinstellelements (11) angeordnet ist, wobei das erste, obere Winkeleinstellelement (11) vorzugsweise scheibenförmig ausgebildet ist.

3. Orthesengelenk (10) nach Anspruch 1 oder 2, **dadurch gekennzeichnet, dass**
- das erste, obere, vorzugsweise scheibenförmige, Winkeleinstellelement (11) wenigstens zwei, bevorzugt vier, an der Unterseite (114) angeordnete, obere Schwenkbegrenzungen (111) umfasst,
- wobei die Schwenkbegrenzungen (111), um einen Winkel (α), welcher durch die jeweiligen Positionen der Schwenkbegrenzungen (111) auf der Unterseite (114) und den Schnittpunkt der Drehachse (D1) mit der Unterseite (114) gebildet wird, zueinander beabstandet sind;
und
- wobei die Schwenkbegrenzungen (111) zueinander derart unterschiedlich ausgebildet sind, dass die Extensionsbewegung des ersten Schwenkhebels (15) durch ein Zusammenwirken mit den verschiedenen Schwenkbegrenzungen (111) in ihrem Bewegungsumfang unterschiedlich begrenzt wird.

4. Orthesengelenk (10) nach einem der Ansprüche 1 bis 3, **dadurch gekennzeichnet, dass** die wenigstens eine untere Schwenkbegrenzung (131) an einer Oberseite (133) des zweiten, unteren Winkeleinstellelements (13) angeordnet ist, wobei das zweite, untere Winkeleinstellelement (13) vorzugsweise scheibenförmig ausgebildet ist.

5. Orthesengelenk (10) nach einem oder mehreren der vorherigen Ansprüche, **dadurch gekennzeichnet, dass**
- das zweite, untere, vorzugsweise scheibenförmige, Winkeleinstellelement (13) wenigstens zwei, bevorzugt vier, an der Oberseite (133) angeordnete, untere Schwenkbegrenzungen (131) umfasst,
- wobei die Schwenkbegrenzungen (131), um einen Winkel (β), welcher durch die jeweiligen Positionen der Schwenkbegrenzungen (131) auf der Oberseite (133) und den Schnittpunkt der Drehachse (D1) mit der Oberseite (133) gebildet wird, zueinander beabstandet sind;
und
- wobei die Schwenkbegrenzungen (131) zueinander derart unterschiedlich ausgebildet sind, dass die Flexionsbewegung des ersten Schwenkhebels (15) durch ein Zusammenwirken mit den verschiedenen Schwenkbegrenzungen (131) in ihrem Bewegungsumfang unterschiedlich begrenzt wird.

6. Orthesengelenk (10) nach einem oder mehreren der vorherigen Ansprüche, **dadurch gekennzeichnet, dass** die Schwenkbegrenzungen (111; 131) als Aussparungen im ersten (11) und/oder zweiten (13) Winkeleinstellelement ausgebildet sind, sodass
- beim ersten Winkeleinstellelement (11) eine Dicke (115) des Winkeleinstellelements (11) von seiner Unterseite (114) in Richtung seiner Oberseite (113) derart verringert ist, dass der erste Schwenkhebel (15) bei einer Schwenkbewegung zum ersten, oberen Winkeleinstellelement (11) hin, nach einem überstrichenen Winkel γ₁ ≥ 0° blockiert wird; und
- beim zweiten Winkeleinstellelement (13) eine Dicke (135) des Winkeleinstellelements (13) von seiner Oberseite (133) in Richtung seiner Unterseite (134) derart verringert ist, dass der erste Schwenkhebel (15) bei einer Schwenkbewegung zum zweiten, unteren Winkeleinstellelement (13) hin, nach einem überstrichenen Winkel γ₂ ≥ 0° blockiert wird.

7. Orthesengelenk (10) nach einem oder mehreren der vorherigen Ansprüche, **dadurch gekennzeichnet, dass**
- an der Unterseite (114) des ersten, oberen Winkeleinstellelements (11) wenigstens ein oberes Einrastmittel (112) angeordnet ist, welches eingerichtet ist, das obere Winkeleinstellelement (11) mit dem Befestigungselement (12) über wenigstens ein, an einer Oberseite (123) des Befestigungselements (12) angeordnetes und korrespondierend zum oberen Einrastmittel (112) ausgebildetes Einrastloch (122), reversibel zu verbinden, sodass eine unbeabsichtigte Drehung des ersten Winkeleinstellelements (11) um die Drehachse (D1) verhindert wird;
und/oder
- dass an der Oberseite (133) des zweiten, unteren Winkeleinstellelements (13) wenigstens ein unteres Einrastmittel (132) angeordnet ist, welches eingerichtet ist, das untere Winkeleinstellelement (13) mit dem Befestigungselement (12) über wenigstens ein, an einer Unterseite (124) des Befestigungselements (12) angeordnetes und korrespondierend zum unteren Einrastmittel (132) ausgebildetes Einrastloch (122), reversibel zu verbinden, sodass eine unbeabsichtigte Drehung des zweiten Winkeleinstellelements (13) um die Drehachse (D1) verhindert wird.

8. Orthesengelenk (10) nach einem oder mehreren der vorherigen Ansprüche, **dadurch gekennzeichnet, dass** am ersten Schwenkhebel (15) wenigstens ein erster Anschlag (151) und wenigstens ein zweiter Anschlag (152) angeordnet sind, welche eingerichtet sind, einen Winkel (δ) der Schwenkbewegung des zweiten Schwenkhebels (16) zu begrenzen,
- wobei die Anschläge (151; 152) vorzugsweise reversibel mit dem ersten Schwenkhebel (15) verbindbar ausgebildet sind.

9. Orthesengelenk (10) nach einem oder mehreren der vorherigen Ansprüche, **dadurch gekennzeichnet, dass** der zweite Schwenkhebel (16) wenigstens einen Schlitten (161) umfasst, welcher eingerichtet ist, den zweiten Schwenkhebel (16) mit einem Verbindungselement (163) zu einer Hand bewegbar zu verbinden, wobei das Verbindungselement (163) dazu vorzugsweise eine Schiene (162) umfasst, welche korrespondierend zu besagtem Schlitten (161) ausgebildet ist.

10. Orthese (1) zur kontrollierten Bewegung und/oder Fixierung einer Hand oder einer Hand und eines Unterarms eines Patienten, **gekennzeichnet durch** ein einstellbares Orthesengelenk (10) nach einem oder mehreren der vorhergehenden Ansprüche 1 bis 9.

## Claims

1. Adjustable orthosis joint (10) for the controlled movement and/or fixation of a hand of a patient, comprising at least
- a first pivot lever (15) for performing an extension and/or flexion movement of the hand;
- a second pivot lever (16), arranged pivotably on the first pivot lever (15), for performing an ulnar and/or radial deviation of the hand;
- wherein the orthosis joint (10) comprises a fastening element (12) for pivotable fastening of the first pivot lever (15);
**characterized in that**
the orthosis joint (10) comprises
- a first, upper angle adjustment element (11), which during use is arranged in relation to the back of a hand above the fastening element (12), rotatably about a rotation axis (D1), on the fastening element (12); and
- a second, lower angle adjustment element (13), which during use is arranged in relation to the back of a hand below the fastening element (12), rotatably about the rotation axis (D1), on the fastening element (12);
- the first angle adjustment element (11) and the second angle adjustment element (13) each comprising at least one pivot limitation (111; 131), which limit the extension and/or flexion movement of the first pivot lever (15) in its range of movement.

2. The orthosis joint (10) as claimed in claim 1, **characterized in that** the at least one upper pivot limitation (111) is arranged on a lower side (114) of the first, upper angle adjustment element (11), the first, upper angle adjustment element (11) preferably being configured in the form of a disk.

3. The orthosis joint (10) as claimed in claim 1 or 2, **characterized in that**
- the first, upper, preferably disk-shaped angle adjustment element (11) comprises at least two, preferentially four upper pivot limitations (111) arranged on the lower side (114),
- the pivot limitations (111) being spaced apart from one another by an angle (α) that is formed by the respective positions of the pivot limitations (111) on the lower side (114) and the point of intersection of the rotation axis (D1) with the lower side (114);
and
- the pivot limitations (111) being configured differently to one another in such a way that the extension movement of the first pivot lever (15) is limited differently in its range of movement by cooperation with the various pivot limitations (111).

4. The orthosis joint (10) as claimed in one of claims 1 to 3, **characterized in that** the at least one lower pivot limitation (131) is arranged on an upper side (133) of the second, lower angle adjustment element (13), the second, lower angle adjustment element (13) preferably being configured in the form of a disk.

5. The orthosis joint (10) as claimed in one or more of the preceding claims, **characterized in that**
- the second, lower, preferably disk-shaped angle adjustment element (13) comprises at least two, preferentially four lower pivot limitations (131) arranged on the upper side (133),
- the pivot limitations (131) being spaced apart from one another by an angle (β) that is formed by the respective positions of the pivot limitations (131) on the upper side (133) and the point of intersection of the rotation axis (D1) with the upper side (133);
and
- the pivot limitations (131) being configured differently to one another in such a way that the flexion movement of the first pivot lever (15) is limited differently in its range of movement by cooperation with the various pivot limitations (131).

6. The orthosis joint (10) as claimed in one or more of the preceding claims, **characterized in that** the pivot limitations (111; 131) are configured as recesses in the first (11) and/or second (13) angle adjustment element, so that
- in the case of the first angle adjustment element (11), a thickness (115) of the angle adjustment element (11) is reduced from its lower side (114) in the direction of its upper side (113) in such a way that the first pivot lever (15) is blocked after a swept angle γ₁ ≥ 0° during a pivot movement toward the first, upper angle adjustment element (11); and
- in the case of the second angle adjustment element (13), a thickness (135) of the angle adjustment element (13) is reduced from its upper side (133) in the direction of its lower side (134) in such a way that the first pivot lever (15) is blocked after a swept angle γ₂ ≥ 0° during a pivot movement toward the second, lower angle adjustment element (13).

7. The orthosis joint (10) as claimed in one or more of the preceding claims, **characterized in that**
- at least one upper latching means (112) is arranged on the lower side (114) of the first, upper angle adjustment element (11), which is adapted to reversibly connect the upper angle adjustment element (11) to the fastening element (12) via at least one latching hole (122) that is arranged on an upper side (123) of the fastening element (12) and is configured so as to correspond with the upper latching means (112), so that accidental rotation of the first angle adjustment element (11) about the rotation axis (D1) is prevented;
and/or
- **in that** at least one lower latching means (132) is arranged on the upper side (133) of the second, lower angle adjustment element (13), which is adapted to reversibly connect the lower angle adjustment element (13) to the fastening element (12) via at least one latching hole (122) that is arranged on a lower side (124) of the fastening element (12) and is configured so as to correspond with the lower latching means (132), so that accidental rotation of the second angle adjustment element (13) about the rotation axis (D1) is prevented.

8. The orthosis joint (10) as claimed in one or more of the preceding claims, **characterized in that** at least one first stop (151) and at least one second stop (152), which are adapted to limit an angle (δ) of the pivot movement of the second pivot lever (16), are arranged on the first pivot lever (15),
- the stops (151; 152) preferably being configured to be reversibly connectable to the first pivot lever (15).

9. The orthosis joint (10) as claimed in one or more of the preceding claims, **characterized in that** the second pivot lever (16) comprises at least one slider (161) which is adapted to movably connect the second pivot lever (16) to a hand connecting element (163), the connecting element (163) for this purpose preferably comprising a rail (162) which is configured so as to correspond with said slider (161).

10. An orthosis (1) for the controlled movement and/or fixation of a hand, or of a hand and a forearm, of a patient, **characterized by** an adjustable orthosis joint (10) as claimed in one or more of the preceding claims 1 to 9.

## Revendications

1. Articulation d'orthèse réglable (10) pour le mouvement et/ou la fixation contrôlés d'une main d'un patient, comprenant au moins
- un premier levier de pivotement (15) pour effectuer un mouvement d'extension et/ou de flexion de la main ;
- un deuxième levier de pivotement (16), agencé de manière pivotante sur le premier levier de pivotement (15), pour effectuer une déviation ulnaire et/ou radiale de la main ;
- l'articulation d'orthèse (10) comprenant
- un élément de fixation (12) pour la fixation pivotante du premier levier de pivotement (15) ;
**caractérisée en ce que**
l'articulation d'orthèse comprend
- un premier élément de réglage d'angle supérieur (11) qui, lors de l'utilisation, est agencé par rapport à un dos de main au-dessus de l'élément de fixation (12) de manière à pouvoir tourner autour d'un axe de rotation (D1) sur l'élément de fixation (12) ; et
- un deuxième élément de réglage d'angle inférieur (13) qui, lors de l'utilisation, est agencé par rapport au dos de main au-dessous de l'élément de fixation (12) de manière à pouvoir tourner autour de l'axe de rotation (D1) sur l'élément de fixation (12) ;
- le premier élément de réglage d'angle (11) et le deuxième élément de réglage d'angle (13) comprenant chacun au moins une limite de pivotement (111 ; 131) qui limite le mouvement d'extension et/ou de flexion du premier levier de pivotement (15) dans son amplitude de mouvement.

2. Articulation d'orthèse (10) selon la revendication 1, **caractérisée en ce que** l'au moins une limite de pivotement supérieure (111) est agencée sur un côté inférieur (114) du premier élément de réglage d'angle supérieur (11), le premier élément de réglage d'angle supérieur (11) étant de préférence réalisé en forme de disque.

3. Articulation d'orthèse (10) selon la revendication 1 ou 2, **caractérisée en ce que**
- le premier élément de réglage d'angle supérieur (11), de préférence en forme de disque, comprend au moins deux, de préférence quatre, limites de pivotement supérieures (111) agencées sur le côté inférieur (114),
- les limites de pivotement (111) étant espacées les unes des autres d'un angle (α) qui est formé par les positions respectives des limites de pivotement (111) sur le côté inférieur (114) et le point d'intersection de l'axe de rotation (D1) avec le côté inférieur (114) ;
et
- les limites de pivotement (111) étant réalisées sous forme différente les unes des autres de telle sorte que le mouvement d'extension du premier levier de pivotement (15) est limité différemment dans son amplitude de mouvement par une coopération avec les différentes limites de pivotement (111).

4. Articulation d'orthèse (10) selon l'une quelconque des revendications 1 à 3, **caractérisée en ce que** l'au moins une limite de pivotement inférieure (131) est agencée sur un côté supérieur (133) du deuxième élément de réglage d'angle inférieur (13), le deuxième élément de réglage d'angle inférieur (13) étant de préférence réalisé en forme de disque.

5. Articulation d'orthèse (10) selon une ou plusieurs des revendications précédentes, **caractérisée en ce que**
- le deuxième élément de réglage d'angle inférieur (13), de préférence en forme de disque, comprend au moins deux, de préférence quatre, limites de pivotement inférieures (131) agencées sur le côté supérieur (133),
- les limites de pivotement (131) étant espacées les unes des autres d'un angle (β) qui est formé par les positions respectives des limites de pivotement (131) sur le côté supérieur (133) et le point d'intersection de l'axe de rotation (D1) avec le côté supérieur (133) ;
et
- les limites de pivotement (131) étant réalisées différemment les unes des autres de telle sorte que le mouvement de flexion du premier levier de pivotement (15) est limité différemment dans son étendue de mouvement par une coopération avec les différentes limites de pivotement (131).

6. Articulation d'orthèse (10) selon une ou plusieurs des revendications précédentes, **caractérisée en ce que** les limites de pivotement (111 ; 131) sont réalisées en forme d'évidements dans le premier (11) et/ou le deuxième (13) élément de réglage d'angle, de telle sorte que
- dans le premier élément de réglage d'angle (11), une épaisseur (115) de l'élément de réglage d'angle (11) est réduite de son côté inférieur (114) en direction de son côté supérieur (113) de telle sorte que le premier levier de pivotement (15) est bloqué lors d'un mouvement de pivotement vers le premier élément de réglage d'angle supérieur (11) après un angle balayé γ₁ ≥ 0° ; et
- dans le deuxième élément de réglage d'angle (13), une épaisseur (135) de l'élément de réglage d'angle (13) est réduite depuis son côté supérieur (133) en direction de son côté inférieur (134) de telle sorte que le premier levier de pivotement (15) est bloqué lors d'un mouvement de pivotement vers le deuxième élément de réglage d'angle inférieur (13) après un angle balayé γ₂ ≥ 0°.

7. Articulation d'orthèse (10) selon une ou plusieurs des revendications précédentes, **caractérisée**
- **en ce qu'**au moins un moyen d'encliquetage supérieur (112) est agencé sur le côté inférieur (114) du premier élément de réglage d'angle supérieur (11), lequel est adapté pour relier de manière réversible l'élément de réglage d'angle supérieur (11) à l'élément de fixation (12) par l'intermédiaire d'au moins un trou d'encliquetage (122) agencé sur un côté supérieur (123) de l'élément de fixation (12) et réalisé en correspondance avec le moyen d'encliquetage supérieur (112), de telle sorte qu'une rotation involontaire du premier élément de réglage d'angle (11) autour de l'axe de rotation (D1) est empêchée ;
et/ou
- **en ce qu'**au moins un moyen d'encliquetage inférieur (132) est agencé sur le côté supérieur (133) du deuxième élément de réglage d'angle inférieur (13), lequel est adapté pour relier de manière réversible l'élément de réglage d'angle inférieur (13) à l'élément de fixation (12) par l'intermédiaire d'au moins un trou d'encliquetage (122) agencé sur un côté inférieur (124) de l'élément de fixation (12) et réalisé en correspondance avec le moyen d'encliquetage inférieur (132), de telle sorte qu'une rotation involontaire du deuxième élément de réglage d'angle (13) autour de l'axe de rotation (D1) est empêchée.

8. Articulation d'orthèse (10) selon une ou plusieurs des revendications précédentes, **caractérisée en ce qu'**au moins une première butée (151) et au moins une deuxième butée (152) sont agencées sur le premier levier de pivotement (15), lesquelles sont adaptées pour limiter un angle (δ) du mouvement de pivotement du deuxième levier de pivotement (16),
- les butées (151 ; 152) étant réalisées de préférence de manière à pouvoir être reliées de manière réversible au premier levier de pivotement (15).

9. Articulation d'orthèse (10) selon une ou plusieurs des revendications précédentes, **caractérisée en ce que** le deuxième levier de pivotement (16) comprend au moins un chariot (161), qui est adapté pour relier de manière mobile le deuxième levier de pivotement (16) par un élément de liaison (163) à une main, l'élément de liaison (163) comprenant à cet effet de préférence une glissière (162) qui est réalisée en correspondance avec ledit chariot (161).

10. Orthèse (1) pour le mouvement et/ou la fixation contrôlés d'une main ou d'une main et d'un avant-bras d'un patient, **caractérisée par** une articulation d'orthèse réglable (10) selon une ou plusieurs des revendications 1 à 9 précédentes.
